(19) Europäisches Patentamt
European Patent Office
Office européen des brevets

(11) **EP 3 086 783 B1**

(12) **EUROPEAN PATENT SPECIFICATION**

(45) Date of publication and mention
of the grant of the patent:
**29.08.2018 Bulletin 2018/35**

(21) Application number: **14824323.1**

(22) Date of filing: **19.12.2014**

(51) Int Cl.:
*A61K 9/70* (2006.01)     *A61K 31/137* (2006.01)

(86) International application number:
**PCT/US2014/071406**

(87) International publication number:
**WO 2015/100153 (02.07.2015 Gazette 2015/26)**

(54) **COMPOSITIONS AND METHODS FOR TRANSDERMAL DELIVERY OF AMPHETAMINE**

ZUSAMMENSETZUNGEN UND VERFAHREN ZUR TRANSDERMALEN VERABREICHUNG VON AMPHETAMIN

COMPOSITIONS ET PROCÉDÉS POUR L'ADMINISTRATION TRANSDERMIQUE D'AMPHÉTAMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **23.12.2013 US 201361919955 P**

(43) Date of publication of application:
**02.11.2016 Bulletin 2016/44**

(73) Proprietor: **Noven Pharmaceuticals, INC.**
**Miami, FL 33186 (US)**

(72) Inventors:
• **NGUYEN, Viet**
**Miami, Florida 33186 (US)**

• **DINH, Steven**
**Miami, Florida 33186 (US)**
• **LIAO, Jun**
**Miami, Florida 33186 (US)**

(74) Representative: **Pohlman, Sandra M.**
**df-mp Dörries Frank-Molnia & Pohlman**
**Patentanwälte Rechtsanwälte PartG mbB**
**Theatinerstrasse 16**
**80333 München (DE)**

(56) References cited:
**WO-A1-2014/066585     WO-A2-2005/042055**
**US-A1- 2006 078 604**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS-REFERENCE TO RELATED APPLICATIONS

This application claims the benefit under 35 U.S.C. § 119(e) to U.S. provisional application 61/919,955, filed December 23, 2013.

BACKGROUND

The present invention relates generally to the transdermal delivery of amphetamine, transdermal drug delivery compositions comprising amphetamine, methods of manufacturing transdermal drug delivery compositions comprising amphetamine, and therapeutic methods using transdermal drug delivery compositions comprising amphetamine, such as may be desired for achieving central nervous system stimulation, such as for the treatment of attention deficit disorder (ADD), attention deficit hyperactivity disorder (ADHD), or for the treatment of narcolepsy.

Many factors influence the design and performance of transdermal drug delivery compositions. These include the individual drugs themselves, the physical and chemical characteristics of the compositions' components and their performance and behavior relative to other components, external and environmental conditions during manufacturing and storage, properties of the application site, the desired rate of drug delivery and therapeutic onset, the desired drug delivery profile, and the intended duration of delivery, among others.

Compositions for the transdermal delivery of amphetamine are known, but there remains a need for compositions that exhibit good stability against the formation of degradation products, such as amphetamine reaction products and degradation products.

Document US 2006/078604 A1 relates to a transdermal drug delivery system for the topical application of active agents contained in polymeric and/or adhesive carrier layers.

Document WO2014/066585 A1 relates to compositions for the transdermal delivery of amphetamine. The compositions comprise a polymer matrix that includes amphetamine or a pharmaceutically acceptable salt or prodrug thereof and a backing layer comprising a polyurethane film layer and a polyester film layer, with a polyurethane adhesive disposed there between.

SUMMARY

The invention relates to compositions for the transdermal delivery of amphetamine in the form of a flexible finite system for topical application, comprising a polymer matrix comprising a polymer and amphetamine or lisdexamfetamine or a pharmaceutically acceptable salt thereof, wherein the composition comprises polyurethane in an amount effective to control the presence of and/or prevent the formation of phenyl acetone in the polymer matrix, wherein the composition comprises polyurethane in an amount effective to control the presence of and/or prevent the formation of phenyl acetone in the polymer matrix, and wherein the polyurethane is included in the polymer matrix and/or is comprised in a layer adjacent the polymer matrix in the form of a polyurethane fiber, particle or powder. In some embodiments, the amphetamine includes amphetamine free base.

In some embodiments, the polyurethane is a reaction product of an isocyanate, such as an isocyanate selected from the group consisting of diphenylmethane diisocyanate, toluene diisocyanate, p-phenylene diisocyanate, naphthalene diisocyanate, hexamethylene diisocyanate, isophorone diisocyanate, dicyclohexylmethane -4,4'-diisocyanate, meta-tetramethylxylylene diisocyanate and transcyclohexane diisocyanate, and a polyol, such as a momomeric polyol, such as glycerin, ethylene glycol, pentaerythritol, or sucrose, or a polymeric polyol, such as natural oil polyols, polyols obtained by polymerization of diols and dicarboxylic acids, polyether polyols, polyols obtained from reacting glycerin with propylene oxide or ethylene oxide or a combination thereof, polycarbonate polyols, polycaprolactone polyols, polybutadiene polyols, and polysulfide polyols, or a combination of a monomeric polyol and a polymeric polyol.

In some embodiments, the composition includes an amount of polyurethane from about 1/100 to about 100 times the amount of amphetamine in the composition.

In some embodiments, the polymer matrix comprises polyurethane, such as polyurethane fibers, particles, or powders.

In some embodiments, the composition further comprises a backing, wherein the backing comprises polyurethane, such as a backing comprising a polyurethane film layer, or a backing comprising a layer comprising a polyurethane additive, such as polyurethane fibers, particles, or powders.

In some embodiments, the composition further comprises a release liner.

Also described are methods of manufacturing a composition as described herein, comprising forming a polymer matrix comprising a polymer and amphetamine or a pharmaceutically acceptable salt or prodrug thereof, wherein the polymer matrix comprises polyurethane.

Also described are methods of manufacturing a composition as described herein, comprising providing a polymer matrix comprising a polymer and amphetamine with a backing comprising polyurethane, such as a backing comprising a polyurethane film layer.

Also described are compositions for use in the transdermal delivery of amphetamine made by any method described herein.

Also described are compositions for use in transdermally administering amphetamine to a subject in need thereof, such as for use in stimulating the central nervous system, treating attention deficit disorder (ADD), treating attention deficit hyperactivity disorder (ADHD), or treating narcolepsy.

Also described are compositions for use in the preparation of a medicament for transdermally administering amphetamine to a subject in need thereof, such as a medicament for stimulating the central nervous system, treating attention deficit disorder (ADD), treating attention deficit hyperactivity disorder (ADHD), or treating narcolepsy.

DETAILED DESCRIPTION

Described herein are transdermal drug delivery compositions comprising amphetamine, methods of making transdermal drug delivery compositions comprising amphetamine and therapeutic methods of using transdermal drug delivery compositions comprising amphetamine. The compositions are provided in a flexible, finite form (e.g., "patch"-type systems) and comprise a polymer matrix that includes a polymer and amphetamine, and also comprise polyurethane. The compositions exhibit good stability, including stability against the formation of degradation products, such as amphetamine reaction products and degradation products, such as phenyl acetone.

DEFINITIONS

Technical and scientific terms used herein have the meanings commonly understood by one of ordinary skill in the art to which the present invention pertains, unless otherwise defined. Reference is made herein to various methodologies known to those of ordinary skill in the art. Any suitable materials and/or methods known to those of ordinary skill in the art can be utilized in carrying out the present invention. However, specific materials and methods are described. Materials, reagents and the like to which reference is made in the following description and examples are obtainable from commercial sources, unless otherwise noted.

As used herein, the singular forms "a," "an," and "the" designate both the singular and the plural, unless expressly stated to designate the singular only.

The term "about" and the use of ranges in general, whether or not qualified by the term about, means that the number comprehended is not limited to the exact number set forth herein, and is intended to refer to ranges substantially within the quoted range while not departing from the scope of the invention. As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

The phrase "substantially free" as used herein means that the described composition (e.g., polymer matrix, etc.) comprises less than about 5%, less than about 3%, or less than about 1% by weight, based on the total weight of the composition at issue, of the excluded component(s).

As used herein "subject" denotes any mammal in need of drug therapy, including humans. For example, a subject may be suffering from or at risk of developing a condition that can be treated or prevented with amphetamine (such as ADD, ADHD. or narcolepsy), or may be taking amphetamine for other purposes.

As used herein, the terms "topical" and "topically" mean application to a skin or mucosal surface of a mammal, while the terms "transdermal" and "transdermal" connote passage through the skin or mucosa (including oral, buccal, nasal, rectal and vaginal mucosa), into systemic circulation. Thus, the compositions described herein may be applied topically to a subject to achieve transdermal delivery of amphetamine.

As used herein, the phrases "therapeutically effective amount" and "therapeutic level" mean that drug dosage or plasma concentration in a subject, respectively, that provides the specific pharmacological effect for which the drug is administered in a subject in need of such treatment. It is emphasized that a therapeutically effective amount or therapeutic level of a drug will not always be effective in treating the conditions/diseases described herein, even though such dosage is deemed to be a therapeutically effective amount by those of skill in the art. For convenience only, exemplary dosages, drug delivery amounts, therapeutically effective amounts and therapeutic levels are provided below with reference to adult human subjects. Those skilled in the art can adjust such amounts in accordance with standard practices as needed to treat a specific subject and/or condition/disease.

As used herein, "active surface area" means the surface area of the drug-containing polymer matrix of the transdermal drug delivery system.

The compositions described herein are in a "flexible, finite form." As used herein, the phrase "flexible, finite form" means a substantially solid form capable of conforming to a surface with which it comes into contact, and capable of maintaining contact so as to facilitate topical application. Such systems in general are known in the art and commercially available, such as transdermal drug delivery patches.

The compositions comprise a drug-containing polymer matrix that releases amphetamine upon application to the skin (or any other surface noted above). The compositions in flexible, finite form also include a backing in addition to the drug-containing polymer matrix layer. In some embodiments, the compositions in flexible, finite form may include a release liner in addition to a drug-containing polymer matrix layer and backing. In some embodiments, the compositions may include one or more other layers.

As used herein, "drug-containing polymer matrix" refers to a polymer composition which contains one or more drugs, such as amphetamine, and a polymer, such as a pressure-sensitive adhesive polymer or a bioadhesive polymer. A polymer is an "adhesive" or "bioadhesive" if it has the properties of adhesiveness per se. Other polymers can function as an adhesive or bioadhesive by the addition of tackifiers, plasticizers, crosslinking agents or other excipients. Thus, in some embodiments, the polymer matrix optionally comprises tackifiers, plasticizers, crosslinking agents or other additives known in the art.

As used herein, the term "pressure-sensitive adhesive" refers to a viscoelastic material which adheres instantaneously to most substrates with the application of very slight pressure and remains permanently tacky. As noted above, a polymer is a pressure-sensitive adhesive polymer if it has the properties of a pressure-sensitive adhesive per se. Other polymers may function as a pressure-sensitive adhesive by admixture with tackifiers, plasticizers or other additives. The term pressure-sensitive adhesive also includes mixtures of different polymers.

In some embodiments, the polymer matrix is a pressure-sensitive adhesive at room temperature and exhibits desirable physical properties, such as good adherence to skin, ability to be peeled or otherwise removed without substantial trauma to the skin, retention of tack with aging, etc. In some embodiments, the polymer matrix has a glass transition temperature ($T_g$), measured using a differential scanning calorimeter, of between about -70 °C. and 0 °C.

In some embodiments, the compositions in flexible, finite form are "monolithic" or "monolayer" systems, such that the drug-containing polymer matrix layer is the only polymeric layer present other than the backing and the release liner, if present. In such embodiments, the polymer matrix functions as both the drug carrier and the means of affixing the system to the skin or mucosa.

*Amphetamine*

Amphetamine (alpha-methylphenethylamine) is a chiral drug. The commercially available oral amphetamine product Adderall® includes several different amphetamine salts, including amphetamine sulfate, amphetamine saccharate, and amphetamine aspartate monohydrate, in an overall ratio of d-amphetamine to l-amphetamine of 3:1. The compositions described herein may be formulated with amphetamine free base or any salt of amphetamine, or any prodrug thereof, or any combinations thereof, and with any isomeric content, and any combinations thereof. In specific embodiments, the compositions comprise d-amphetamine. In further specific embodiments the amphetamine component consists essentially of d-amphetamine (e.g., it contains no more than trace amounts of other amphetamine species). In still further specific embodiments the amphetamine component consists of d-amphetamine. In other specific embodiments, the composition comprises a prodrug of d-amphetamine, such as lisdexamfetamine, in the free base or any salt form, such as lisdexamfetamine dimesylate.

In addition to the salts mentioned above, exemplary suitable pharmaceutically acceptable salts of amphetamine are salts of weak inorganic and organic acids, and quaternary ammonium salts. These include without limitation, salts with acids such as sulfuric, phosphoric, hydrochloric, hydrobromic, hydriodic, sulfamic, citric, lactic, maleic, malic, succinic, tartaric, cinnamic, acetic, benzoic, gluconic, or ascorbic acid, or quaternary ammonium salts with organic esters of sulfuric, hydrohalic, or aromatic sulfonic acids, such as methyl chloride, methyl bromide, ethyl chloride, propyl chloride, butyl chloride, isobutyl chloride, benzylchloride, benzyl bromide, phenethyl bromide, naphthymethyl chloride, dimethyl sulfate, methyl benzenesulfonate, ethyl toluenesulfonate, ethylene chlorohydrin, propylene chlorobydrin, allyl bromide, methylallyl bromide or crotyl bromide esters.

The compositions described herein include a therapeutically effective amount of amphetamine and/or pharmaceutically acceptable salt(s) and/or prodrug(s) thereof. Generally, the amount of amphetamine is from about 1% to about 50%, including from about 5% to about 40%, such as from about 10% to about 20% by weight, based on the total dry weight of the polymer matrix. In specific embodiments, the polymer matrix comprises about 15% by weight amphetamine, based on the total dry weight of the polymer matrix. In other specific embodiments, the polymer matrix comprises about 10% by weight amphetamine, based on the total dry weight of the polymer matrix. In other specific embodiments, the polymer matrix comprises about 20% by weight amphetamine, based on the total dry weight of the polymer matrix.

In accordance with any of the embodiments described herein, the composition may include from about 5 to about 30 mg of amphetamine base or an equivalent amount of a pharmaceutically acceptable salt or prodrug thereof, including about 5, 10, 15, 20, 25, or 30 mg of amphetamine base or equivalent amount of salt or prodrug.

Amphetamine may react with certain components that typically are used in the manufacture of transdermal drug delivery compositions, such as components that contain a reactive moiety such as an acetyl moiety, a vinyl acetate moiety, an acyl halide moiety, a carbonate ester moiety, or a carboxyl moiety. Examples of such components include polymers, adhesives, excipients, solvents, or other components that contain an acetyl moiety, a vinyl acetate moiety, an acyl halide moiety, a carbonate ester moiety, a carboxyl moiety, or an ester moiety. For example, amphetamine may react with such a component to form an amphetamine reaction product, such as N-acetyl amphetamine or phenyl acetone.

The formation of an amphetamine reaction product is undesirable from at least two perspectives. First, the formation of an amphetamine reaction product reduces the amount of amphetamine present in the composition, and so may reduce the amount of therapeutically effective amphetamine species, which may impair the efficacy of the compositions. Second, as a general principle, it is desirable to minimize the formation of reaction products and degradants in pharmaceutical products. For example, the U.S. Food and Drug Administration typically requires characterization and qualification of such substances when present in an amount greater than or equal to about 0.5%.

In some embodiments, the compositions and methods described herein address this problem by formulating and/or manufacturing amphetamine such that the formation of amphetamine reaction products is reduced, minimized, or avoided. Thus, in some embodiments, the polymer matrix comprises a polymer that is free of vinyl acetate moieties. In other embodiments, the polymer matrix is free of components that include reactive moieties, such as acetyl moieties, vinyl acetate moieties, acyl halide moieties, carbonate ester moieties, carboxyl moieties, and ester moieties. Additionally or alternatively, the polymer matrix is made using processing solvents that are free of such reactive moieties. In some embodiments, additionally or alternatively, the compositions and methods described herein address this problem by formulating the amphetamine in a flexible, finite system that includes polyurethane, in the polymer matrix or in an adjacent layer, as described in more detail below.

### *Polymer Matrix*

In accordance with some embodiments, the compositions described herein comprise a polymer matrix that comprises, consists essentially of, or consists of amphetamine and/or pharmaceutically acceptable salt(s) thereof and at least one polymer.

Acrylate monomers which can be used include acrylic acid, methacrylic acid, methyl acrylate, methyl methacrylate, butyl acrylate, butyl methacrylate, hexyl acrylate, hexyl methacrylate, 2-ethylbutyl acrylate, 2-ethylbutyl methacrylate, isooctyl acrylate, isooctyl methacrylate, 2-ethylhexyl acrylate, 2-ethylhexyl methacrylate, decyl acrylate, decyl methacrylate, dodecyl acrylate, dodecyl methacrylate, tridecyl acrylate, tridecyl methacrylate, and octyl acrylamide. In specific embodiments, the non acid-functional acrylic polymer includes methacrylate (methyl acrylate) monomers and 2-ethylhexyl acrylate monomers. In other specific embodiments the non acid-functional acrylic polymer includes methacrylate (methyl acrylate) monomers, 2-ethylhexyl acrylate monomers, butyl acrylate monomers and amide-group containing monomers such as octylacrylamide.

Suitable acrylic polymers which are commercially available include those sold by Henkel North America under the Duro-Tak® brand name such as Durotak® 87-2353, 73-9257, 73-9259, 73-9261, 87-2097, 87-2510, 87-4098, 87-9301A, 87-900A, 87-901A, 87-9082, 87-9085, 87-9088, and by Cytec Industries Inc. under the Gelva® GMS brand name, such as Gelva® GMS 3071, GMS 3067, GMS 3087, and GMS 3235. Other suitable acrylic polymers are known in the art. See, e.g., the non acid-functional acrylic polymers described in Satas, "Acrylic Adhesives, HANDBOOK OF PRESSURE- SENSITIVE ADHESIVE TECHNOLOGY, 2nd ed. , pp. 396-456 (D. Satas, ed.), Van Nostrand Reinhold, N. Y. (1989); "Acrylic and Methacrylic Ester Polymers," POLYMER SCIENCE AND ENGINEERING, Vol. 1, 2nd ed., pp 234-268, John Wiley & Sons, (1984).

In some embodiments, the polymer matrix additionally or alternatively comprises a rubber-based polymer, such as a rubber-based adhesive polymer. Examples of suitable rubber-based polymers include polyisobutylene polymers and styrene-isoprene-styrene block copolymers.

Polyisobutylene polymers suitable for use in a polymer matrix of transdermal drug delivery compositions are known, and include those sold by BASF under the Oppanol® brand, such as Oppanol® B11. In some embodiments, the polymer matrix comprises two or more polyisobutylene polymers of different molecular weights. In accordance with these embodiments, the relative amounts of polyisobutylene polymers can be selected and tailored to produce a product with satisfactory physical and pharmacokinetic properties.

Styrene-isoprene-styrene block copolymers suitable for use in a polymer matrix of transdermal drug delivery compositions are known, and include those sold by Kraton under the Kraton® brand, such as Kraton® D111 KT.

In some embodiments, the polymer matrix additionally or alternatively comprises a silicone-based polymer, such as a silicone-based adhesive polymer. The term "silicone-based" polymer as used herein is used interchangeably with the terms siloxane, polysiloxanc, and silicones, as is conventional in the art. The silicone-based polymer may be a pressure-sensitive adhesive, such as a polysiloxane adhesive prepared by cross-linking an elastomer, typically a high molecular weight polydiorganosiloxane, with a resin, to produce a three-dimensional siloxane structure, via a condensation reaction in an appropriate organic solvent. The ratio of resin to elastomer can be adjusted in order to modify the physical properties of polysiloxane adhesives. Sobieski, et al., "Silicone Pressure Sensitive Adhesives," Handbook of Pressure-Sensitive Adhesive Technology. 2nd ed., pp. 508-517 (D. Satas, ed.), Van Nostrand Reinhold, New York (1989). Further details and examples of silicone pressure-sensitive adhesives which are useful are described in the following U.S. Patents. Nos. 4,591,622; 4,584,355; 4,585,836; and 4,655,767. Suitable silicone pressure-sensitive adhesives are commercially available and include the silicone adhesives sold under the trademarks BIO-PSA® by Dow Corning Corporation, Medical Products, Midland, Mich. (such as -2685, -3027, -3122, -4101, -4102,-4203, -4301, -4302, -4303, -4401-4403, -4501, -4503, -4602, -4603 and -4919). In some embodiments, the silicone polymer is a capped (or amine-compatible) polysiloxane. A "capped" polysiloxane polymer is one which has been chemically treated to reduce or eliminate the silicone-bonded hydroxyl content, such as by substitution with a hydrocarbon radical such as a methyl group. Illustrative examples of capped polysiloxanes include those described in U.S. Patent No. Re. 35,474 and U.S. Patent No. 6,337,086 and which are commercially available from Dow Corning Corporation under their Bio-PSA X7-4100, -4200 and -4300 product series. In some embodiments, the silicone polymer has a high silanol content (e.g., a silicone-bonded hydroxyl content of greater than about 13,000), such as the silicone-based adhesives that are commercially available from Dow Corning Corporation under their BIO-PSA 7-4400, -4500, and -4600 product series.

When the polymer matrix comprises more than one polymer, each polymer can be included in any amount. The relative amounts of each polymer can be selected and tailored to achieve desired physical properties (e.g., strength, tackiness, peel strength, etc.), desired drug solubility/drug loading, and/or desired pharmacokinetic properties (e.g., onset and duration of drug delivery and drug delivery profile, etc.).

*Polyurethane*

As noted above, in some embodiments the amphetamine compositions described herein comprise polyurethane. The polyurethane may be included in the polymer matrix with the amphetamine, or may be provided in another layer of the system, such as in a layer adjacent the drug-containing polymer matrix layer, such as in the backing or another layer, such as an adhesive layer or rate controlling membrane adjacent the drug-containing polymer matrix layer. The polyurethane may be provided as a component of the drug-containing layer or other layer, such as in fiber, particle, or powder form, or may be provided as a layer, such as a polyurethane film layer,

Surprisingly, it has been found that formulating amphetamine in a transdermal drug delivery composition with polyurethane controls the presence of and/or inhibits the formation of amphetamine reaction/degradation products in the composition, such as phenyl acetone, e.g., controls the presence of and/or inhibits the formation of amphetamine reaction/degradation products such as phenyl acetone in the drug-containing polymer matrix. Without being bound by theory it is believed that the polyurethane may interact with the amphetamine in such a way that prevents the formation of phenyl acetone, which may result from an oxidation reaction.

Suitable polyurethanes can be synthesized by reacting an isocyanate with a polyol. For example, the isocyanate can be one or more selected from the group consisting of diphenylmethane diisocyanate, toluene diisocyanate, p-phenylene diisocyanate, naphthalene diisocyanate, hexamethylene diisocyanate, isophorone diisocyanate, dicyclohexylmethane - 4,4'-diisocyanate, meta-tetramethylxylylene diisocyanate and transcyclohexane diisocyanate. The polyol can be any monomeric or polymeric polyol, or combinations thereof. Examples of monomeric polyols inclue glycerin, ethylene glycol, pentaerythritol, sucrose, and the like. Examples of polymeric polyols include natural oil polyols, polyols obtained by polymerizing diols and dicarboxylic acids (e.g., diethylene glycol and phthalic acid), polyether polyols such as polyethylene glycol, polypropylene glycol, poly(tetramethylene ether)glycol, polyols obtained by reacting glycerin with propylene oxide or ethylene oxide or a combination thereof, polycarbonate polyols, polycaprolactone polyols, polybutadiene polyols, polysulfide polyols, and the like.

As noted above, in some embodiments, polyurethane is included in the polymer matrix with the amphetamine. Additionally, or alternatively, polyurethane may be included in another layer, such as another layer adjacent the amphetamine-containing polymer matrix layer, such as in the backing or in any other layer that may be present, such as an adhesive layer or a rate controlling layer, such as a rate controlling membrane. In any of these embodiments, the polyurethane may be provided as an adhesive, such as in film form, or as an additive, such as in powder, particle or fiber form.

In some embodiments, the transdermal drug delivery compositions include an amount of polyurethane that is effective to control the presence of and/or prevent the formation of phenyl acetone in the composition, e.g., an amount of polyurethane that is effective to control the presence of and/or prevent the formation of phenyl acetone in the drug-containing polymer matrix. In some embodiments, the composition includes an amount of polyurethane from 1/100 to 100 times the amount of amphetamine present, such as from 1/100 to 100 times the amount of amphetamine free base present in the composition. This amount can be provided in one layer, such as in the amphetamine-containing polymer matrix layer or in a backing layer, or can be distributed across two or more layers, such as in both the amphetamine-containing polymer matrix layer and a backing layer, and/or in one or more other layers that may be present.

### Other Components

As noted above, the polymer matrix of the compositions described herein optionally may further comprise other components typically used in a transdermal drug delivery composition, such as tackifiers, plasticizers, crosslinking agents or other excipients known in the art. In some embodiments, any such components that are present do not include a reactive moiety, such as an acetyl moiety, a vinyl acetate moiety, an acyl halide moiety, a carbonate ester moiety, a carboxyl moiety, or an ester moiety.

The polymer matrix may further comprise various tackifying agents, thickeners, fillers, and other additives or components known for use in transdermal drug delivery systems. These optional components include tackifying agents such as aliphatic hydrocarbons, mixed aliphatic and aromatic hydrocarbons, aromatic hydrocarbons, substituted aromatic hydrocarbons, hydrogenated esters, hydrogenated hydrocarbon resins, styrene-isobutylene-styrene block copolymers, polyterpenes, silicone fluid, mineral oil and hydrogenated wood rosins; binders, such as lecithin which "bind" the other ingredients; rheological agents (thickeners) containing silicone, such as fumed silica, reagent grade sand, precipitated silica, amorphous silica, colloidal silicon dioxide, fused silica, silica gel, quartz and particulate siliceous materials commercially available as Syloid®, Cabosil®, Aerosil®, and Whitelite®, such as for enhancing the uniform consistency or continuous phase of the composition or coating. Other additives and excipients include diluents, stabilizers, fillers, clays, buffering agents, biocides, humectants, anti-irritants, antioxidants, preservatives, plasticizing agents, cross-linking agents, flavoring agents, colorants, pigments and the like. Such substances can be present in any amount sufficient to impart the desired properties to the composition. As noted above, in some embodiments, any such components that are present do not include a reactive moiety, such as an acetyl moiety, a vinyl acetate moiety, an acyl halide moiety, a carbonate ester moiety, a carboxyl moiety.

Such additives or excipients are typically used in amounts totaling up to 50%, including from about 0.1% to about 30%, by weight based on the dry weight of the polymer matrix. As noted above, in some embodiments, any such components that are present do not include a reactive moiety, such as an acetyl moiety, a vinyl acetate moiety, an acyl halide moiety, a carbonate ester moiety, a carboxyl moiety.

Amphetamine base does not generally require a penetration enhancer. Thus, in some embodiments, the polymer matrix is substantially free of penetration enhancers. In some embodiments, the polymer matrix is free of penetration enhancers. By "free of penetration enhancers" is meant that the composition is formulated without penetration enhancers, such that at most only trace amounts are present as impurities or contaminants.

### Transdermal Drug Delivery Systems

In embodiments where the polymer matrix comprises a pressure-sensitive adhesive or bioadhesive, the polymer matrix can serve as an adhesive portion of the transdermal drug delivery system (e.g., a reservoir device), or can serve as one or more layers of a multi-layer system. Alternatively, a polymer matrix comprising a pressure-sensitive adhesive or bioadhesive with drug dissolved or dispersed therein can constitute a monolithic transdermal drug delivery system. In embodiments where the polymer matrix does not comprise an adhesive, but instead, for example, comprises a polymeric drug reservoir, it can be used in combination with one or more adhesive layers, or with a surrounding adhesive portion, as is well known to those skilled in the art. Additionally or alternatively, in some embodiments, the transdermal drug delivery system may comprise one or more additional layers, such as a rate controlling layer, such as a rate controlling membrane.

In some embodiments, a transdermal drug delivery system consists essentially of the polymer matrix layer. By "consists essentially of the polymer matrix layer" means that the system does not contain any other layers that affect drug delivery, such as an additional rate-controlling polymer layer, rate-controlling membrane, or drug reservoir layer. It will be understood, however, that the system that consists essentially of the polymer matrix layer may comprise a backing and/or release liner.

The transdermal drug delivery system may be of any shape or size suitable for transdermal application.

### Backing

The compositions in flexible, finite form comprise a polymer matrix, such as described above, and a backing. The backing is impermeable to the drug (e.g., impermeable to the amphetamine) and is adjacent one face of the polymer matrix. (By "impermeable" to the drug is meant that no substantial amount of drug loss through the backing layer is observed.) The backing protects the polymer matrix from the environment and prevents loss of the drug and/or release of other components to the environment during use.

As noted above, in some embodiments the backing comprises polyurethane. In accordance with such embodiments, the polyurethane may be provided as a polyurethane film, e.g., as a layer of the backing layer and/or as an additive in a backing layer comprised of another material (e.g., as a powder, particle, or fiber). In some embodiments the backing comprises polyurethane in an amount effective to control the presence of and/or prevent the formation of phenyl acetone in the system. In some embodiments the backing comprises polyurethane in an amount less than such an effective amount, but the system as a whole includes polyurethane in such an effective amount. In some embodiments, the backing includes an amount of polyurethane from 1/100 to 100 times the amount of amphetamine present in the system, such as from 1/100 to 100 times the amount of amphetamine free base present in the system. In some embodiments, the backing comprises polyurethane in one layer, while in other embodiments the backing comprises polyurethane in more than one layer. In any of these embodiments, the system is configured such that at least some of the polyurethane can come into contact with amphetamine present in the system, such as providing at least some of the polyurethane in a layer adjacent the drug-containing polymer matrix.

In accordance with specific embodiments, the backing layer comprises a polyurethane layer comprised of a polyether aromatic polyurethane film, such as ST-1882-82 available from Stevens Urethane (Easthampton, MA), which has a specific gravity of about 1.14 and a melting point range of about 284 to 320 F (about 140 to 160 C), or ST-1882P-82 available from Stevens Urethane (Easthampton, MA) which has a specific gravity of about 1.14 and a melting point range of about 290 to 330 F (about 143 to 166 C). Other polyurethane films can be used, such as those available from 3M, such as 3M CoTran™ 9701 (St. Paul, MN).

In embodiments where the backing comprises a polyurethane film layer, the backing may include one or more other layers, on the other side of the polyurethane film layer from the drug-containing layer. This is because a polyurethane film may not possess the structural integrity typically desired in a backing, such as the physical integrity and moisture impermeability that may be desired for a flexible, finite system comprising amphetamine. In these embodiments, the one or more other layers may comprise one or more other materials known in the art and used for such purposes, such as another polymer film (such as a polyester film, an aluminum-coated polyester film, an ethylene vinyl acetate film, an acrylonitrile-methyl acrylate copolymer film, an ethylene acid polymer film, etc.), paper, fabric, aluminum foil, and the like. For example the 3M CoTran™ 9701 polyurethane film is provided commercially with a paper layer. In some embodiments, the backing includes a polyurethane film layer and an ethylene vinyl acetate film layer. In some embodiments, the backing include a polyurethane film layer and an aluminum-coated polyester film. In some embodiments, the backing include a polyurethane film layer and an acrylonitrile-methyl acrylate copolymer film (e.g. BAREX ®, available from INOES USA LLC). In some embodiments, the backing include a polyurethane film layer and an ethylene acid polymer-based film (e.g. Surlyn®, available from DuPont). In some embodiments where the backing layer comprises a polyurethane film layer, the backing layer does not also include a polyester film layer.

### Release Liner

The compositions in flexible, finite form may further comprise a release liner, typically located adjacent the opposite face of the system as the backing layer. When present, the release liner is removed from the system prior to use to expose the polymer matrix layer prior to topical application. Materials suitable for use as release liners are well-known in the art and commercially available, such as polyester release liners, including coated polyester release liners, such as siliconized or fluoro-coated polyester release liners. In specific embodiments, the release liner is a silicone-coated polyester release liner, such as those available from Loparex Inc. (Iowa City, IA), including those sold as product D 3.0 CL PET 4000E/000, having a thickness of about 3 mils (+/- 10%). In other specific embodiments, the release liner is a fluoropolymer-coated polyester release liner, such as those available from 3M (St. Paul, MN), including those sold as Scotchpak™ 9744, having a thickness of about 2.9 mils (+/- 0.2 mils).

### Methods of Manufacture

The compositions described herein can be prepared by methods known in the art. As one step, the polymer matrices described herein can be prepared by methods known in the art, such as blending (mixing) the polymer component(s) in powder or liquid form with an appropriate amount of drug in the presence of an appropriate solvent, such as a volatile organic solvent, optionally with other excipients. To form a final product, the drug/polymer/solvent mixture may be cast onto a release liner, followed by evaporation of the volatile solvent(s), for example, at room temperature, slightly elevated temperature, or by a heating/drying step, to form the drug-containing polymer matrix on a release liner. A preformed backing may be applied to form a final product. Appropriate size and shape delivery systems are die-cut from the roll material and then pouched. As noted above, in accordance with some embodiments, polyurethane is included in one or more layers.

The order of steps, the amount of the ingredients, and the amount and time of agitation or mixing may be important process variables which will depend on the specific polymers, active agents, solvents and/or cosolvents, and optional excipients used in the composition, but these factors can be adjusted by those skilled in the art. The order in which each method step is performed can be changed if needed without detracting from the invention.

In accordance with any of the embodiments of the compositions described herein, the coat weight of the polymer matrix can be, in some embodiments, from about 3 mg/cm$^2$ to about 10 mg/cm$^2$, based on the active surface area of the polymer matrix. Exemplary coat weights include about 3 mg/cm$^2$, about 4 mg/cm$^2$, about 5 mg/cm$^2$, about 5.5 mg/cm$^2$, about 6 mg/cm$^2$, about 6.5 mg/cm$^2$, about 7 mg/cm$^2$, about 7.5 mg/cm$^2$, about 8 mg/cm$^2$, about 8.5 mg/cm$^2$, about 9 mg/cm$^2$, about 9.5 mg/cm$^2$, and about 10 mg/cm$^2$. In specific embodiments the coat weight of the polymer matrix is about 6.0 to about 8.0 mg/cm$^2$, including about 7.0 mg/cm$^2$, based on the active surface area of the of the polymer matrix.

In accordance with any of the embodiments of the compositions described herein, the amphetamine can be present, in some embodiments, in an amount from about 0.5 mg/cm$^2$ to about 3 mg/cm$^2$, based on the active surface area of the of the polymer matrix, such as about 1 mg/cm$^2$, including about 1.05 mg/cm$^2$, based on the active surface area of the of the polymer matrix. Other exemplary amounts include about 0.75 mg/cm$^2$, 0.8 mg/cm$^2$, 0.9 mg/cm$^2$, 1.0 mg/cm$^2$, 1.05 mg/cm$^2$, 1.1 mg/cm$^2$, 1.2 mg/cm$^2$, and 1.25 mg/cm$^2$, 1.5 mg/cm$^2$, 2.0 mg/cm$^2$, 2.5 mg/cm$^2$, and 3.0 mg/cm$^2$.

In accordance with any of the embodiments of compositions described herein, the size of the final product (e.g., the composition in the form of a the flexible, final system) may be, in some embodiments, in the range of from about 2 cm$^2$ to about 60 cm$^2$, including from about 5 cm$^2$ to about 30 cm$^2$, including about 4.75 cm$^2$, 5 cm$^2$, 10 cm$^2$, 15 cm$^2$, 20 cm$^2$, 25 cm$^2$, and 30 cm$^2$. In specific embodiments, the final product includes an amount of amphetamine of about 1.05 mg/cm$^2$, such that, for example, a 5 cm$^2$ flexible, finite system includes about 5.25 mg amphetamine, and flexible, finite systems having a size of 10 cm$^2$, 15 cm$^2$, 20 cm$^2$, 25 cm$^2$, and 30 cm$^2$ have proportionate amounts of amphetamine.

### Methods of Use

The compositions described herein are for use in transdermal delivery of amphetamine, including for use in the treatment of attention deficit disorder, attention deficit hyperactivity disorder, and/or narcolespy. In such embodiments, a composition comprising a therapeutically effective amount of amphetamine as described herein is for use in topical application to a subject in need thereof.

In general, the compositions achieve transdermal delivery of amphetamine over a period of time of about 8 to 10 hours, including a period of time of about 9 hours, although the composition may remain on the application site for a longer period of time.

The compositions described herein achieve a transdermal flux of amphetamine (and/or one or more pharmaceutically acceptable salt(s) thereof) that is sufficient to have a therapeutic effect. As used herein, "flux" (also called "permeation rate") is defined as the absorption of a drug through skin or mucosal tissue, and is described by Fick's first law of diffusion:

$$J = -D \, (dCm/dx)$$

where J is the flux in g/cm$^2$/sec, D is the diffusion coefficient of the drug through the skin or mucosa in cm2/sec and dCm/dx is the concentration gradient of the drug across the skin or mucosa.

The following specific examples are included as illustrative of the compositions described herein. These examples are in no way intended to limit the scope of the invention.

### Example 1

The formation of phenyl acetone from transdermal drug delivery systems comprised of the same release liner, the same polymer matrix layer, and different backings is assessed. The polymer matrix layer comprises 15% w/w amphetamine free base and 85% w/w pressure sensitive adhesive. The release liner is a silicone-coated polyester release liner. The backings are set forth in the table below.

The systems are stored for one month at 40°C and phenyl acetone content is determined by HPLC. Results are shown in the table, with phenyl acetone content reported as w/w of the amphetamine present.

| Backing Layer | Polyurethane Film (ST-1882P-82 1.5 mil) | Polyurethane Film laminated with Polyester Film | Polyester Film | Ethylene Vinyl Acetate Film | Aluminum Vapor-Coated Polyester Film |
|---|---|---|---|---|---|
| Phenyl Acetone Content after 1 Month at 40 °C | 0.1% | 0.2% | 2.4% | 3% | 2.5% |

The results show that formulating amphetamine in a composition with a polyurethane backing layer adjacent

EP 3 086 783 B1

the drug-containing polymer matrix layer controls the presence of and/or inhibits the formation of phenyl acetone.

### Example 2

The ability of a backing comprising a polyurethane layer to reduce the phenyl acetone present in a transdermal drug delivery system is compared to that of BHT (butylated hydroxytoluene, an antioxidant) when formulated in the polymer matrix. Transdermal drug delivery systems comprises amphetamine free base (AMPH), pressure sensitive adhesive (PSA) and, optionally BHT, were prepared as outlined below and phenyl acetone content was assessed shortly after manufacture by HPLC.

Results are shown in the table, with phenyl acetone content reported as w/w of the amphetamine present.

| Backing | Polyurethane Film laminated with Polyester Film | Polyester/ Ethylene Vinyl Acetate Film | Polyester/ Ethylene Vinyl Acetate Film |
|---|---|---|---|
| Matrix | 15% AMPH<br>85% PSA | 15% AMPH<br>84% PSA<br>1% BHT | 15% AMPH<br>85% PSA |
| Phenyl Acetone Content | 0.2% | 0.8 % | 1.4% |

The results show that a backing comprising a polyurethane film layer adjacent the drug-containing polymer matrix layer controls the presence of and/or inhibits the formation of phenyl phenyl acetone to a greater extent than formulating the drug-containing polymer matrix with BHT, although BHT does exhibit some impact on phenyl acetone content as compared to a matrix formulated without BHT and provided with a non-polyurethane backing.

### Example 3

The ability of backing comprising a polyurethane layer to reduce the phenyl acetone present in a transdermal drug delivery system is assessed as compared to that of a backing comprising BHT. Transdermal drug delivery systems were prepared as outlined below and phenyl acetone content was assessed shortly after manufacture by HPLC.

Results are shown in the table, with phenyl acetone content reported as w/w of the amphetamine present.

| Backing | Polyurethane Film (ST-1882P-82, 1.5 mil) | Polyester/ Ethylene Vinyl Acetate Film | Polyester/ Ethylene Vinyl Acetate Film with about 3.6 $\mu$g BHT/10cm$^2$ |
|---|---|---|---|
| Matrix | 15% AMPH<br>85% PSA | 15% AMPH<br>85% PSA | 15% AMPH<br>85% PSA |
| Phenyl Acetone | Not Detected | 0.4 % | 0.4% |

The results show that a backing comprising a polyurethane film layer adjacent the drug-containing polymer matrix layer controls the presence of and/or inhibits the formation of phenyl acetone, whereas the presence of BHT in the backing does not (compare the results with the two polyester/ethylene vinyl acetate backings, which differ by the presence of BHT).

## Claims

1. A composition for the transdermal delivery of amphetamine in the form of a flexible finite system for topical application, comprising a polymer matrix comprising a polymer and amphetamine or lisdexamfetamine or a pharmaceutically acceptable salt thereof, wherein the composition comprises polyurethane in an amount effective to control the presence of and/or prevent the formation of phenyl acetone in the polymer matrix, and wherein the polyurethane is included in the polymer matrix and/or is comprised in a layer adjacent the polymer matrix in the form of a polyurethane fiber, particle or powder.

**2.** The composition of claim 1, wherein the amphetamine includes amphetamine free base.

**3.** The composition of claim 1, wherein the polyurethane is a reaction product of an isocyanate and a polyol, optionally wherein the polyurethane is a reaction product of (i) an isocyanate selected from the group consisting of diphenylmethane diisocyanate, toluene diisocyanate, p-phenylene diisocyanate, naphthalene diisocyanate, hexamethylene diisocyanate, isophorone diisocyanate, dicyclohexylmethane -4,4'-diisocyanate, meta-tetramethylxylylene diisocyanate and transcyclohexane diisocyanate and (ii) a polyol.

**4.** The composition of claim 3, wherein the polyol is a momomeric polyol, optionally wherein the momomeric polyol is selected from the group consisting of glycerin, ethylene glycol, pentaerythritol, and sucrose.

**5.** The composition of claim 3, wherein the polyol is a polymeric polyol, optionally wherein the polymeric polyol is selected from the group consisting of natural oil polyols, polyols obtained by polymerization of diols and dicarboxylic acids, polyether polyols, polyols obtained from reacting glycerin with propylene oxide or ethylene oxide or a combination thereof, polycarbonate polyols, polycaprolactone polyols, polybutadiene polyols, and polysulfide polyols.

**6.** The composition of claim 3, wherein the polyol is a combination of a monomeric polyol and a polymeric polyol.

**7.** The composition of claim 1, wherein the composition includes an amount of polyurethane from about 1/100 to about 100 times the amount of amphetamine in the composition.

**8.** The composition of claim 1, wherein the polymer matrix comprises polyurethane.

**9.** The composition of claim 1, further comprising a backing and/or a release liner.

**10.** The composition of claim 1, wherein the polyurethane is comprised in a layer adjacent the polymer matrix in the form of a polyurethane fiber, particle or powder.

**11.** A method of manufacturing a composition as claimed in any one of claims 1-10, comprising forming a polymer matrix comprising a polymer and amphetamine or lisdexamfetamine, or a pharmaceutically acceptable salt thereof, wherein the polymer matrix comprises polyurethane.

**12.** A method of manufacturing a composition as claimed in any one of claims 1-10, comprising providing a polymer matrix comprising a polymer and amphetamine or lisdexamfetamine, or a pharmaceutically acceptable salt thereof with a layer adjacent the polymer matrix that comprises a polyurethane fiber, particle or powder.

**13.** A composition made by a method according to any one of claims 11 or 12 for use in transdermal delivery of amphetamine.

**14.** A composition according to any one of claims 1-10 or 13, for use in transdermally administering amphetamine to a subject in need thereof.

**15.** A composition according to any one of claims 1-10 or 13, for use in stimulating the central nervous system, treating attention deficit disorder (ADD), treating attention deficit hyperactivity disorder (ADHD), or treating narcolepsy.

**Patentansprüche**

**1.** Zusammensetzung zur transdermalen Applikation von Amphetamin in Form eines flexiblen, endlichen Systems zur örtlichen Anwendung, umfassend eine Polymermatrix, umfassend ein Polymer und Amphetamin oder Lisdexamfetamin oder ein pharmazeutisch akzeptables Salz davon, wobei die Zusammensetzung Polyurethan in einer Menge umfasst, die zum Kontrollieren des Vorhandenseins von und/oder Verhindern der Bildung von Phenylaceton in der Polymermatrix wirksam ist, und wobei das Polyurethan in der Polymermatrix enthalten ist und/oder in einer an die Polymermatrix angrenzenden Schicht in Form einer Polyurethanfaser, eines Polyurethanteilchens oder eines Polyurethanpulvers eingeschlossen ist.

**2.** Zusammensetzung nach Anspruch 1, wobei das Amphetamin freie Amphetaminbase einschließt.

**3.** Zusammensetzung nach Anspruch 1, wobei das Polyurethan ein Reaktionsprodukt von einem Isocyanat und einem Polyol ist, wobei wahlweise das Polyurethan ein Reaktionsprodukt von (i) einem Isocyanat, ausgewählt aus der Gruppe bestehend aus Diphenylmethandiisocyanat, Toluoldiisocyanat, p-Phenylendiisocyanat, Naphthalendiisocyanat, Hexamethylendiisocyanat, Isophorondiisocyanat, Dicyclohexylmethan-4,4'-diisocyanat, meta-Tetramethylxylylendiisocyanat und Transcyclohexandiisocyanat, und (ii) einem Polyol ist.

**4.** Zusammensetzung nach Anspruch 3, wobei das Polyol ein monomeres Polyol ist, wobei wahlweise das monomere Polyol ausgewählt ist aus der Gruppe bestehend aus Glycerin, Ethylenglycol, Pentaerythrit und Saccharose.

**5.** Zusammensetzung nach Anspruch 3, wobei das Polyol ein polymeres Polyol ist, wobei wahlweise das polymere Polyol ausgewählt ist aus der Gruppe bestehend aus Naturölpolyolen, Polyolen, die durch Polymerisation von Diolen und Dicarbonsäuren gewonnen werden, Polyetherpolyolen, Polyolen, die aus der Umsetzung von Glycerin mit Propylenoxid oder Ethylenoxid oder einer Kombination davon gewonnen werden, Polycarbonatpolyolen, Polycaprolactonpolyolen, Polybutadienpolyolen und Polysulfidpolyolen.

**6.** Zusammensetzung nach Anspruch 3, wobei das Polyol eine Kombination aus einem monomeren Polyol und einem polymeren Polyol ist.

**7.** Zusammensetzung nach Anspruch 1, wobei die Zusammensetzung eine Menge an Polyurethan einschließt, die von etwa 1/100- bis etwa 100-mal die Menge an Amphetamin in der Zusammensetzung beträgt.

**8.** Zusammensetzung nach Anspruch 1, wobei die Polymermatrix Polyurethan umfasst.

**9.** Zusammensetzung nach Anspruch 1, ferner umfassend eine Unterlage und/oder einen Release-Liner.

**10.** Zusammensetzung nach Anspruch 1, wobei das Polyurethan in einer an die Polymermatrix angrenzenden Schicht in Form einer Polyurethanfaser, eines Polyurethanteilchens oder eines Polyurethanpulvers eingeschlossen ist.

**11.** Verfahren zur Herstellung einer Zusammensetzung nach einem der Ansprüche 1 bis 10, umfassend das Bilden einer Polymermatrix, umfassend ein Polymer und Amphetamin oder Lisdexamfetamin oder ein pharmazeutisch akzeptables Salz davon, wobei die Polymermatrix Polyurethan umfasst.

**12.** Verfahren zur Herstellung einer Zusammensetzung nach einem der Ansprüche 1 bis 10, umfassend das Versehen einer Polymermatrix, umfassend ein Polymer und Amphetamin oder Lisdexamfetamin oder ein pharmazeutisch akzeptables Salz davon, mit einer an die Polymermatrix angrenzenden Schicht, die eine Polyurethanfaser, ein Polyurethanteilchen oder ein Polyurethanpulver umfasst.

**13.** Zusammensetzung, hergestellt durch ein Verfahren nach einem der Ansprüche 11 oder 12, zur Verwendung bei der transdermalen Applikation von Amphetamin.

**14.** Zusammensetzung nach einem der Ansprüche 1 bis 10 oder 13 zur Verwendung bei der transdermalen Verabreichung von Amphetamin an einen Patienten, der dessen bedarf.

**15.** Zusammensetzung nach einem der Ansprüche 1 bis 10 oder 13 zur Verwendung bei der Stimulierung des zentralen Nervensystems, der Behandlung von Aufmerksamkeitsdefizitstörung (ADD), der Behandlung von Aufmerksamkeitsdefizitstörung mit Hyperaktivität (ADHD) oder der Behandlung von Narkolepsie.

**Revendications**

**1.** Composition pour l'administration transdermique d'amphétamine sous forme d'un système fini souple destiné à l'administration topique, comprenant une matrice polymère comprenant un polymère et de l'amphétamine ou de la lisdexamphétamine ou un sel pharmaceutiquement acceptable correspondant, la composition comprenant du polyuréthane en une quantité efficace pour réguler la présence et/ou empêcher la formation de phénylacétone dans la matrice polymère et le polyuréthane étant inclus dans la matrice polymère et/ou étant compris dans une couche adjacente à la matrice polymère sous forme de fibre, de particule ou de poudre de polyuréthane.

**2.** Composition selon la revendication 1, l'amphétamine incluant la base libre d'amphétamine.

**3.** Composition selon la revendication 1, le polyuréthane étant un produit de réaction d'un isocyanate et d'un polyol, éventuellement, le polyuréthane étant un produit de réaction de (i) un isocyanate choisi dans le groupe constitué par le diisocyanate de diphénylméthane, le diisocyanate de toluène, le diisocyanate de p-phénylène, le diisocyanate de naphtalène, le diisocyanate d'hexaméthylène, le diisocyanate d'isophorone, le 4,4'-diisocyanate de dicyclohexylméthane, le diisocyanate de méta-tétraméthylxylylène et le diisocyanate de transcyclohexane et de (ii) un polyol.

**4.** Composition selon la revendication 3, le polyol étant un polyol monomère, éventuellement, le polyol monomère étant choisi dans le groupe constitué par le glycérol, l'éthylèneglycol, le pentaérythritol et le saccharose.

**5.** Composition selon la revendication 3, le polyol étant un polyol polymère, éventuellement, le polyol polymère étant choisi dans le groupe constitué par les polyols d'huile naturelle, les polyols obtenus par polymérisation de diols et d'acides dicarboxyliques, les polyéther-polyols, les polyols obtenus par la réaction de glycérol avec de l'oxyde de propylène ou de l'oxyde d'éthylène ou avec une combinaison correspondante, les polycarbonate-polyols, les polycaprolactame-polyols, les polybutadiène-polyols et les polysulfure-polyols.

**6.** Composition selon la revendication 3, le polyol étant une combinaison d'un polyol monomère et d'un polyol polymère.

**7.** Composition selon la revendication 1, la composition incluant une quantité de polyuréthane représentant environ 1/100 à environ 100 fois la quantité d'amphétamine dans la composition.

**8.** Composition selon la revendication 1, la matrice polymère comprenant du polyuréthane.

**9.** Composition selon la revendication 1, comprenant en outre un support et/ou une protection détachable.

**10.** Composition selon la revendication 1, le polyuréthane étant compris dans une couche adjacente à la matrice polymère sous forme de fibre, de particule ou de poudre de polyuréthane.

**11.** Procédé de production d'une composition selon l'une quelconque des revendications 1-10, comprenant la formation d'une matrice polymère comprenant un polymère et de l'amphétamine ou de la lisdexamphétamine ou un sel pharmaceutiquement acceptable correspondant, la matrice polymère comprenant du polyuréthane.

**12.** Procédé de production d'une composition selon l'une quelconque des revendications 1-10, comprenant le fait de munir une matrice polymère, comprenant un polymère et de l'amphétamine ou de la lisdexamphétamine ou un sel pharmaceutiquement acceptable correspondant, d'une couche adjacente à la matrice polymère qui comprend une fibre, une particule ou une poudre de polyuréthane.

**13.** Composition produite par un procédé selon l'une quelconque des revendications 11 ou 12 destinée à être utilisée dans l'administration transdermique d'amphétamine.

**14.** Composition selon l'une quelconque des revendications 1-10 ou 13, destinée à être utilisée dans l'administration transdermique d'amphétamine à un sujet qui en a besoin.

**15.** Composition selon l'une quelconque des revendications 1-10 ou 13, destinée à être utilisée dans la stimulation du système nerveux central, dans le traitement du trouble déficitaire de l'attention (TDA), dans le traitement du trouble déficitaire de l'attention avec hyperactivité (TDAH) ou dans le traitement de la narcolepsie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61919955 A
- US 2006078604 A1
- WO 2014066585 A1
- US 4591622 A
- US 4584355 A
- US 4585836 A
- US 4655767 A
- US RE35474 E
- US 6337086 B

### Non-patent literature cited in the description

- Acrylic Adhesives. **SATAS.** HANDBOOK OF PRES- SURE- SENSITIVE ADHESIVE TECHNOLOGY. Van Nostrand Reinhold, 1989, 396-456
- Acrylic and Methacrylic Ester Polymers. POLYMER SCIENCE AND ENGINEERING. John Wiley & Sons, 1984, vol. 1, 234-268
- Silicone Pressure Sensitive Adhesives. **SOBIESKI et al.** Handbook of Pressure-Sensitive Adhesive Technology. Van Nostrand Reinhold, 1989, 508-517